# EUROPÄISCHE PATENTSCHRIFT

(11) **EP 2 388 582 B1**
(45) Veröffentlichungstag und Bekanntmachung des Hinweises auf die Patenterteilung: **22.08.2012**
(21) Anmeldenummer: 11163103.2
(22) Anmeldetag: 20.04.2011
(51) Int. Cl.: G01N 33/28, G01N 11/00, F02D 41/14, F01L 9/02

(54) **Verfahren sowie Steuereinrichtung zur Ermittlung einer Viskositäts-Kenngröße eines Motoröls**
Method and control device for determining a viscosity parameter of a motor oil
Procédé et dispositif de commande pour l'établissement d'une grandeur caractéristique de viscosité d'une huile de moteur

(30) Priorität: 17.05.2010 DE 102010020755
(43) Veröffentlichungstag der Anmeldung: 23.11.2011
(73) Patentinhaber: Schaeffler Technologies AG & Co. KG, 91074 Herzogenaurach (DE)
(72) Erfinder: Traversa, Piergiacomo, 90451 Nürnberg (DE)

(56) Entgegenhaltungen:
- DE-A1- 19 741 164
- JP-A- 2004 092 593
- US-A1- 2004 244 742
- US-A1- 2006 052 216
- L. BERNARD ET AL.: "Elektrohydraulische Ventilsteuerung mit dem "MultiAir"-Verfahren", MOTORTECHNISCHE ZEITSCHRIFT MTZ, Dezember 2009 (2009-12), Seiten 892-898, XP001525896,

## Beschreibung

### Gebiet der Erfindung

Die Erfindung betrifft ein Verfahren sowie eine Steuereinrichtung zur Ermittlung einer Viskositäts-Kenngröße eines Motoröls in einem Verbrennungsmotor mit einer hydraulischen Steuerung der Gaswechselventile.

### Hintergrund der Erfindung

Bei modernen Kraftfahrzeugen werden heutzutage aus Gründen der Schadstoffminimierung sowie der Verbrauchsreduzierung die sogenannten Gaswechselventile, also die Einlass- und/ oder Auslassventile für den Kraftfahrzeugmotor, lastabhängig gesteuert. Hierbei finden unterschiedliche Systeme Einsatz. Allen Systemen ist gemeinsam, dass die Schließ- und/ oder Öffnungszeiten der Gaswechselventile in Relation zur Kurbelwellenposition (Drehwinkel) lastabhängig verändert werden. Hierzu werden auch hydraulische Steuerungssysteme eingesetzt, bei denen mit Hilfe eines Hydraulikfluids, nämlich das Motoröl, eine Änderung der Schließ- und/ oder Öffnungszeiten des Gaswechselventils herbeigeführt wird.

So ist beispielsweise aus der EP 1 544 419 A1 ein erstes hydraulisches Steuerungssystem zu entnehmen, nämlich ein hydraulischer Nockenwellenversteller. Bei einem derartigen System wird mit Hilfe des Hydraulikfluids der Phasenwinkel zwischen der Kurbelwelle und der Nockenwelle eines Kraftfahrzeug-Motors verändert. Dies wird durch die variable Befüllung von Druckkammern einer Verstellvorrichtung erzielt. Zur Ansteuerung und Befüllung bzw. Entleerung der Druckkammern sind üblicherweise Steuerventile vorgesehen, die insbesondere als Magnetventile ausgebildet sind.

Aus dem Artikel "Elektrohydraulische Ventilsteuerung mit dem "MultiAir"-Verfahren" aus der motortechnischen Zeitschrift MTZ 12/2009 ist ein alternatives hydraulisches Steuersystem für die Ansteuerung der Gaswechselventile zu entnehmen. Bei dieser elektrohydraulischen Ventilsteuerung ist vorgesehen, dass die Bewegung der Nockenwelle über die Hydraulikflüssigkeit auf ein jeweiliges Gaswechselventil übertragen wird. Zur Steuerung ist ein insbesondere als Magnetventil ausgebildetes Steuer- oder Schaltventil vorgesehen. Im geschlossenen Zustand ist die Nockenwelle mit dem jeweiligen Gaswechselventil über ein sogenanntes hydraulisches Gestänge verbunden, so dass das Gaswechselventil einem Nocken der Nockenwelle zwangsweise folgt. Durch auch teilweises Öffnen des Schaltventils kann das Hydraulikfluid in einen Ausgleichs- oder Druckraum entweichen, so dass das Gaswechselventil von der Nockenbewegung entkoppelt ist. Hierdurch besteht die Möglichkeit, den Öffnungszeitpunkt, den Schließzeitpunkt sowie den Hub des Gaswechselventils innerhalb einer durch die Bewegung des Nockens vorgegebenen Hüllkurve zu variieren. Diese Variation kann zylinderselektiv erfolgen.

Im Hinblick auf die bei modernen Verbrennungsmotoren geforderte hohe Effizienz bei gleichzeitig geringer Schadstoffemission ist eine exakte Ansteuerung der Gaswechselventile von entscheidender Bedeutung. Bei hydraulischen Systemen wirkt sich die Art der verwendeten Hydraulikflüssigkeit, also insbesondere die Qualität des eingesetzten Motoröls, auf den Betrieb wesentlich aus. Insbesondere ist die hydraulische Steuerung in ihrer Wirkungsweise empfindlich gegenüber Schwankungen der Viskosität des eingesetzten Öls. Betriebsbedingt treten solche Schwankungen aufgrund unterschiedlicher Öltemperaturen auf. Wie aus dem Artikel "Elektrohydraulische Ventilsteuerung mit dem "MultiAir"-Verfahren" hervorgeht, wurden die temperaturabhängigen Ölviskositätsschuwankungen bisher in einem modellbasierten Steueralgorithmus berücksichtigt, der die Messwerte eines Öltemperatursensors berücksichtigt.

In der US 2004/0244742 A1 wird ein Verfahren zur Ermittlung einer Viskositäts-Kenngröße eines Motoröls in einem Verbrennungsmotor mit einer hydraulischen Steuerung der Gaswechselventile vorgeschlagen. Zur Ermittlung der Viskositäts-Kenngröße ist ein nicht näher spezifizierter Viskositätssensor eingesetzt. Weiter wird in der JP 2004092593 A zur hydraulischen Steuerung von Gaswechselventilen vorgeschlagen, die Viskosität der Hydraulikflüssigkeit über deren Temperatur zu ermitteln, wobei die Schaltgeschwindigkeiten der Gaswechselventile berücksichtigt werden. Bei einer hydraulisch betätigten Treibstoffeinspritzvorrichtung entsprechend der DE 197 41 164 wird die Viskosität des als Hydraulikflüssigkeit eingesetzten Motoröls über den Abgabedruck einer Ölpumpe und der Motordrehzahl bestimmt. Auch aus der US 2006/0052216 A1 ist zu einer hydraulischen Steuerung von Gaswechselventilen bekannt, die Viskosität des eingesetzten Öls zu berücksichtigen.

Eine temperaturabhängige und damit indirekte Bestimmung weist Nachteile auf und berücksichtigt beispielsweise keine Alterung des Motoröls oder einen Verschleiß der hydraulischen Komponenten.

### Aufgabe der Erfindung

Ausgehend hiervon liegt der Erfindung die Aufgabe zugrunde, ein verbessertes Verfahren sowie eine verbesserte Steuereinrichtung zur direkten Ermittlung einer Viskositäts-Kenngröße für ein Motoröl anzugeben, wobei insbesondere auch Alterungs- und Verschleißeffekte berücksichtigt werden können.

### Lösung der Aufgabe

Die Aufgabe wird gemäß der Erfindung gelöst durch ein Verfahren sowie eine Steuereinrichtung zur Ermittlung einer Viskositäts-Kenngröße eines Motoröls in einem Verbrennungsmotor mit einer hydraulischen Steuerung der Gaswechselventile, insbesondere mit einer elektrohydraulischen Steuerung der Gaswechselventile, wie sie sich beispielsweise ergibt aus dem Artikel "Elektrohydraulische Ventilsteuerung mit dem MultiAir-Verfahren", motortechnische Zeitschrift MTZ 12/2009. Bei dem Verfahren ist vorgesehen, dass eine Vergleichsgröße, die mit einer gemessenen Sauerstoffkonzentration in einem Abgas des Verbrennungsmotors korreliert ist, als ein Maß für die Viskositäts-Kenngröße, insbesondere als Maß für die Viskosität selbst herangezogen wird. Grundsätzlich wird daher aus der gemessenen Sauerstoffkonzentration ein Maß für die Viskosität abgeleitet oder bestimmt.

Diese Ausgestaltung beruht auf der Überlegung, dass insbesondere bei einer elektrohydraulischen Ansteuerung der Gaswechselventile die im Abgas enthaltene Sauerstoffkonzentration - bei gegebenen Betriebsparametern des Verbrennungsmotors - von der Viskosität des eingesetzten Motoröls abhängt. Bei einem elektrohydraulischen Steuersystem für die Ansteuerung der Gaswechselventile wird durch eine gezielte Ansteuerung eines Steuer- oder Schaltventils die Bewegung des Gaswechselventils von der Bewegung der Nockenwelle entkoppelt. Üblicherweise ist bei geöffnetem Schaltventil die ansonsten steife hydraulische Verbindung zwischen dem Nocken der Nockenwelle und dem Gaswechselventil aufgehoben. Das Gaswechselventil geht üblicherweise federkraftbetätigt in seinen Ausgangszustand, den geschlossenen Zustand über. Dieses selbsttätige, von der Bewegung des Nockens entkoppeltes Schließen des Gaswechselventils wird nachfolgend auch als ballistische Phase bezeichnet, da in diesem Bereich das Gaswechselventil nicht mehr zwangsgeführt ist. Während der ballistischen Phase arbeitet die Federkraft gegen eine Reibungskraft, die maßgeblich von der Viskosität des Motoröls bestimmt ist. Dies bedeutet, dass der Verlauf der ballistischen Phase und damit die Zeitdauer bis zum Schließen des Gaswechselventils von der Viskosität des Motoröls abhängt. Gleichzeitig bestimmt der Schließzeitpunkt auch die Menge der für den jeweiligen Verbrennungsvorgang angesaugten Luft. Weist z.B. das Öl eine sehr hohe Viskosität auf, verlangsamt sich der Schließvorgang des Gaswechselventils und der Schließzeitpunkt verschiebt sich nach hinten, so dass insgesamt im Vergleich zu einem Motoröl mit einer geringeren Viskosität eine größere Luftmenge in den Zylinder angesaugt wird. Dies äußert sich - da die Menge des eingespritzten Brennstoffs (Benzin/Diesel) durch eine Einspritzsteuerung vorgegeben ist - in unterschiedlichen Sauerstoffkonzentrationen im Abgas.

Aufgrund dieser Zusammenhänge ist daher die im Abgas enthaltene Sauerstoffkonzentration zumindest ein indirektes Maß für die aktuelle Viskosität des Motoröls. Im Grunde wird daher mit dem beschriebenen Verfahren der Verlauf der ballistischen Phase insbesondere in Korrelation zu einer durch die Bewegung des Nockens vorgegebenen Hüllkurve ausgewertet.

Die Sauerstoffkonzentration wird bei Kraftfahrzeugen für die so genannte λ-Regelung herangezogen, um das Verhältnis von Brennstoff zu Sauerstoff in gewünschter Weise einzustellen. Für den Wert λ = 1 ist ein stöchiometrisches Verhältnis eingestellt, bei λ<1 wird der Motor mit einem Überschuss an Brennstoff (fettes Gemisch) und bei λ>1 wird der Verbrennungsmotor mit Luftüberschuss (mageres Gemisch) betrieben. Für die λ-Regelung wird eine so genannte λ-Sonde eingesetzt, die den Rest-Sauerstoffgehalt im Abgas insbesondere durch Vergleich mit dem Sauerstoffgehalt in der Umgebungsluft ermittelt. Das vorliegende Verfahren nutzt daher bereits vorhandene Installationen und Informationen bei einem Kraftfahrzeug aus. Für die Bestimmung der Viskositäts-Kenngröße des Motoröls sind daher keine zusätzlichen Komponenten erforderlich. Die Ermittlung der Viskosität erfolgt daher - ausgehend von einer bestehenden λ-Regelung - ausschließlich steuerungstechnisch und damit softwarebasiert.

Die Vergleichsgröße, die als Maß für die Viskositäts-Kenngröße herangezogen wird, ist in einer Variante vorzugsweise die gemessen Sauerstoffkonzentration selbst. Alternativ hierzu ist die Vergleichsgröße eine hieraus abgeleitete Größe oder der Verlauf einer hieraus abgeleiteten Größe, wie beispielsweise ein von der λ-Regelung abgegebenes Steuersignal, welches auf charakteristische Änderungen überwacht wird.

Die Viskositäts-Kenngröße ist vorzugsweise die Viskosität selbst. Alternativ besteht jedoch auch die Möglichkeit, die Vergleichsgröße als indirektes Maß für die Viskositätsgröße für weitergehende Auswertungen heranzuziehen.

Gemäß einer zweckdienlichen Weiterbildung wird die λ-Regelung zur Ermittlung der Viskositäts-Kenngröße ausgewertet. Bei diesem Konzept wird daher nicht der absolute Wert der Sauerstoffkonzentration ausgewertet, sondern das Regelverhalten der λ-Regelung. Dies beruht auf der Überlegung, dass beispielsweise infolge von Alterungserscheinungen die Viskosität des Motoröls ansteigt und dass - im Vergleich zum vorhergehenden Zustand - über die λ-Regelung es zu einer fehlerhaften Einstellung kommt und der im Abgas gemessene Sauerstoffgehalt von dem - bei unveränderter Öleigenschaft - erwarteten Sauerstoffgehalt abweicht. Dieser "Fehler" wird von der λ-Regelung korrigiert. Vorzugsweise wird diese Korrektur, die letztendlich eine Korrektur der Sauerstoffkonzentration im Abgas ist, zur Ermittlung der Viskositäts-Kenngröße herangezogen. Die λ-Reglung wird daher insbesondere auf typische Abweichungen überwacht, die von Änderungen der Viskosität des Motoröls hervorgerufen sein können.

Gemäß einer zweckdienlichen Weiterbildung ist in einer Steuereinrichtung eine Korrelation zwischen der Vergleichsgröße und der Viskositäts-Kenngröße hinterlegt, beispielsweise im Rahmen eines Kennlinienfeldes. Die Korrelation wurde hierbei insbesondere im Vorfeld in Testreihen und Testversuchen ermittelt, so dass zur Bestimmung der Viskositäts-Kenngröße im laufenden Betrieb lediglich noch ein Vergleich der erfassten Vergleichsgröße mit den hinterlegten Werten erforderlich ist.

Aufgrund der unterschiedlichsten Bedingungen, unter denen der Verbrennungsmotor betrieben werden kann, ist diese Korrelation bzw. sind die Kennlinienfelder in Abhängigkeit zumindest eines Betriebsparameters des Verbrennungsmotors vorzugsweise in Abhängigkeit einer Vielzahl von Betriebsparametern hinterlegt. Derartige Betriebsparameter des Verbrennungsmotors sind insbesondere dessen Temperatur (und damit die Temperatur des Motoröls), seine Motordrehzahl, die aufgrund der aktuellen Lastanforderung gewählte Einspritzmenge, etc.

Vorzugsweise ist weiterhin vorgesehen, dass die Korrelation vorzugsweise ergänzend auch in Abhängigkeit einer Kenngröße des Motoröls hinterlegt ist. Eine derartige Kenngröße ist insbesondere die Viskositätsklasse, beispielsweise gemäß der SAE-Klassifizierung. Diese Klassifizierung charakterisiert die Warmlaufeigenschaften bei vergleichsweise hohen Umgebungstemperaturen (Sommeröl) und die Kaltlaufeigenschaften bei vergleichsweise kalten Umgebungstemperaturen (Winteröl). Es sind daher vorzugsweise für unterschiedliche Motoröl-Klassifizierungen Kennlinienfelder hinterlegt, diese wiederum in Abhängigkeit der Betriebsparameter des Verbrennungsmotors.

Darüber hinaus ist in bevorzugter Weiterbildung vorgesehen, dass nach Hersteller bzw. Marken individualisierte Motoröle in den Kennlinienfeldern hinterlegt sind. Die Kennlinienfelder geben dabei beispielsweise die Abhängigkeit der Restsauerstoffkonzentration im Abgas gegenüber einem Steuersignal der λ-Regelung oder auch direkt gegenüber der Viskosität für vorgegebene Betriebsparameter und Kenngrößen des Motoröls wieder.

In bevorzugter Weiterbildung wird die Viskositäts-Kenngröße zylinderspezifisch ermittelt. Das bedeutet, dass für jeden Zylinder des Verbrennungsmotors oder zumindest für Gruppen von Zylindern des Verbrennungsmotors die Vergleichsgröße ermittelt und hieraus die Viskositäts-Kenngröße abgeleitet wird. Dies wird insbesondere bei derartigen Kraftfahrzeugen eingesetzt, die eine zylinderspezifische λ-Regelung bzw. Sauerstoffmessung vorsehen. Damit wird die Auswertung und Analyse des Sauerstoffgehalts im Abgas und damit die Ermittlung der Viskositäts-Kenngröße robuster und genauer und weniger fehleranfällig.

Die ermittelte Viskositäts-Kenngröße wird in bevorzugten Ausgestaltungen alternativ oder in Kombination für unterschiedliche abgeleitete Maßnahmen und Auswertungen herangezogen. Neben der Auswertung der aktuell ermittelten Viskositäts-Kenngröße kann dabei auch die Auswertung der Änderung der Viskositäts-Kenngröße herangezogen werden.

Gemäß einer bevorzugten ersten Alternative wird ein Maß für die eingesetzte Ölqualität ermittelt, insbesondere auch, welcher SAE-Klassifizierung das Motoröl genügt. Durch Messungen oder Ermittlungen der Vergleichsgröße bei verschiedenen Arbeitspunkten des Verbrennungsmotors, also bei bestimmten Temperaturen Drehzahlen, etc. lässt sich aus den verschiedenen Messpunkten ein Verlauf der Vergleichsgröße beispielsweise in Abhängigkeit der Temperatur etc. ermitteln. Aus diesem Verlauf kann durch Vergleich mit einem hinterlegten Kennlinienfeld bestimmt werden, was für ein Motoröl eingesetzt ist. In gleicher Weise wird in bevorzugter Ausgestaltung auch ein unzulässig eingesetztes Motoröl erkannt. Durch die Abgabe eines entsprechenden Warnsignals können damit Motorschäden verhindert werden.

Gemäß einer bevorzugten weiteren Auswertung ist vorgesehen, dass die Viskositäts-Kenngröße auf eine abrupte Änderung hin überwacht wird, um beispielsweise zu erkennen, dass ein Motorölwechsel vorgenommen wurde. In diesem Fall wird vorzugsweise von der Steuereinrichtung ein Kenner oder eine Markierung gesetzt, die den Motorölwechsel kennzeichnet und identifiziert. Dieser Kenner wird vorzugsweise auch zur Bestimmung des Ölwechselintervalls herangezogen. Grundsätzlich wird die Viskositäts-Kenngröße in einer weiteren bevorzugten Ausgestaltung herangezogen, um den Ölwechselintervall zu bestimmen. Aufgrund der laufenden Messung wird nämlich eine Verschlechterung des Motoröls rechtzeitig erkannt. Eine derartige Verschlechterung gegenüber einem ursprünglich errechneten Ölwechselintervall kann beispielsweise bei Kurzstreckenfahrten auftreten.

Schließlich wird in besonders zweckdienlicher Ausgestaltung anhand der ermittelten Viskositäts-Kenngröße Einfluss auf die Ventilsteuerung genommen. Zum einen werden beispielsweise bei einer erfassten Alterung des Motoröls, d.h. bei einer erfassten Erhöhung der Viskosität, die Zeitpunkte für die Ansteuerung des Schaltventils variiert und den neuen Gegebenheiten angepasst. Hierdurch wird eine möglichst exakte Gasventilsteuerung entsprechend den jeweiligen Anforderungen gewährleistet. Durch diese Maßnahme wird daher erreicht, dass die ballistische Phase zu dem gewünschten Zeitpunkt beendet ist und das Gaswechselventil wieder in seiner Schließstellung ist. Vorzugsweise wird die gesamte elektrohydraulische Steuerung auf Grundlage der ermittelten Kenngröße für die Viskosität vorgenommen, und nicht - wie bisher üblich - anhand der aktuellen Öltemperatur. Die gesamte Steuerung der Gaswechselventile beruht daher auf den tatsächlichen Zustand des Öls.

Weiterhin ist in bevorzugter Ausgestaltung vorgesehen, dass ein Warnsignal abgegeben wird, wenn die ermittelte Ölviskosität einen störungsfreien Betrieb des Motors nicht zulässt. So kann sogar ein Starten des Motors unterbunden werden, beispielsweise im Falle von extrem niedrigen Außentemperaturen und einer zu hohen Viskosität und einer damit einhergehenden mangelnden Schmierung des Motors. Aufgrund des in einem vorhergehenden Betriebszyklus ermittelten Ölzustands lässt sich nämlich eine minimale Außentemperatur bestimmen, die nicht unterschritten werden darf.

Die Ermittlung der Viskositäts-Kenngröße erfolgt vorzugweise kontinuierlich oder in Abständen während des Betriebs des Verbrennungsmotors und damit mehr oder weniger kontinuierlich. Hierbei kann vorgesehen sein, dass die Ermittlung der Viskositäts-Kenngröße nur ein oder wenige Male während eines Betriebszyklus des Verbrennungsmotors (also zwischen Starten und Stoppen des Motors) erfolgt.

### Kurze Beschreibung der Zeichnung

Ein Ausführungsbeispiel der Erfindung wird im Folgenden anhand der Figuren näher erläutert. Es zeigen:
- Fig. 1: in einer schematisch und stark vereinfachten ausschnittsweisen Darstellung die prinzipielle Funktionsweise einer elektrohydraulischen Steuerung eines Gaswechselventils in einem Kraftfahrzeugmotor,
- Fig. 2: in vereinfachter Darstellung den typischen Verlauf eines Steuersignals für ein Schaltventil der elektrohydraulischen Steuerung und den hierdurch bedingten Verlauf des Gaswechselventils mit einer ballistischen Phase im Vergleich zu dem Verlauf, den das Gaswechselventil bei Zwangskopplung mit den Nocken der Nockenwelle einnehmen würde, und
- Fig. 3: einen beispielhaften Verlauf eines Lambda-Korrekturwerts in Abhängigkeit eines Phasenwinkels φ, der ein Maß für Phasenlage zwischen der Schließstellung des Gaswechselventils und einer Drehposition der Kurbelwelle ist.

In den Figuren sind gleich wirkende Teile mit den gleichen Bezugszeichen versehen.

### Ausführliche Beschreibung der Zeichnung

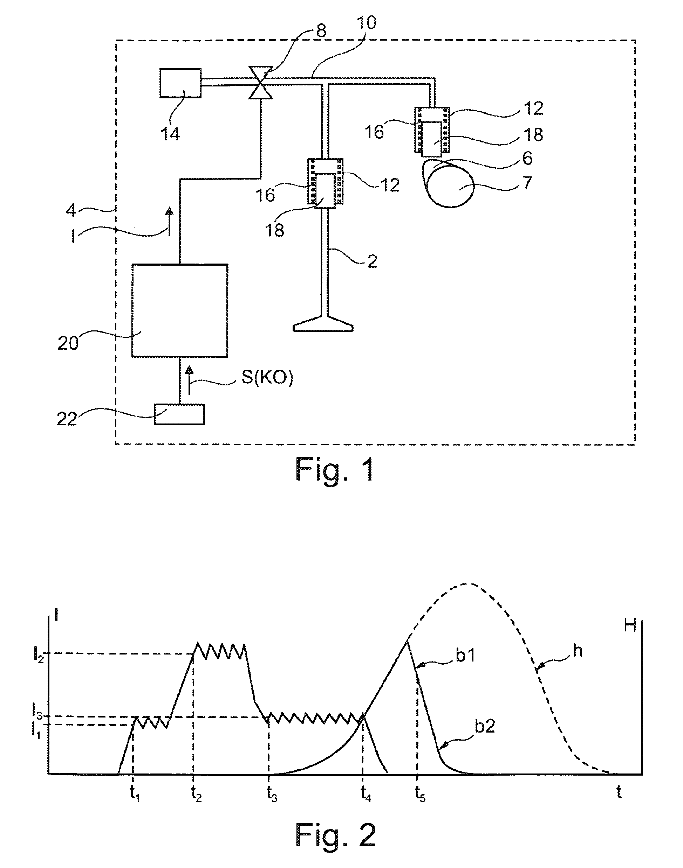

Ein elektrohydraulisches Steuerungssystem zur hydraulischen Steuerung eines Gaswechselventils 2 in einem in Fig. 1 durch gestrichelte Umrandung angedeuteten Kraftfahrzeug 4 umfasst ein hydraulisches System, welches eine Bewegung eines Nockens 6 einer Nockenwelle 7 auf ein jeweiliges Gaswechselventil 2 mittels einer Hydraulikflüssigkeit, nämlich dem Motoröl überträgt. Die elektrohydraulische Ventilsteuerung ist an sich bekannt. Wesentliches Merkmal ist ein insbesondere als Magnetventil 8 ausgebildetes Schaltventil, welches in eine Hydraulikleitung 10 geschaltet ist. Die Hydraulikleitung 10 ist über einen Hydraulikzylinder 12 einerseits mit dem Nocken 6 und andererseits mit dem Gaswechselventil 2 verbunden. Über das Magnetventil 8 lässt sich die Hydraulikleitung 10 zu einem Ausgleichs- oder Druckraum 14 absperren. Innerhalb der Hydraulikzylinder 12 sind Kolben 18 beispielsweise gegen die Kraft einer Feder 16 beweglich gelagert. Bei einer Rotation des Nockens 6 folgt der Kolben 18 des zugeordneten Hydraulikzylinders 12 der Nockenbewegung. Bei geschlossenem Magnetventil 8 wirkt das hydraulische System nach Art eines hydraulischen Gestänges, so dass der Kolben 18 in dem dem Gaswechselventil 2 zugeordneten Hydraulikzylinder 12 der Bewegung des Nockens 6 unmittelbar folgt.

Durch Öffnen des Magnetventils 8 kann das Öl in den Druckraum 14 ausweichen, so dass die Bewegung des Gaswechselventils 2 von der Bewegung des Nockens 6 entkoppelt ist.

Das Magnetventil 8 ist mit einer Steuer- oder Auswerteeinheit 20 verbunden. Die Steuereinheit 20 ist beispielsweise in der Motorsteuerung integriert. Über die Steuereinheit 20 wird das Magnetventil 8 mit einem Steuersignal beaufschlagt. Im Ausführungsbeispiel ist dies ein Erregerstrom I für eine Magnetspule des Magnetventils 8.

Die Steuereinheit 20 ist weiterhin mit einer sogenannten λ-Sonde 22 verbunden. Die λ-Sonde 22 ist in einer hier nicht näher dargestellten Abgasleitung, üblicherweise in einem Abgaskrümmer, angeordnet und misst in an sich bekannter Weise den (Rest-) Sauerstoffgehalt KO im Abgas des Verbrennungsmotors des Kraftfahrzeugs 4. Die λ-Sonde 22 gibt ein entsprechendes Sauerstoff-Messsignal S (KO) an die Steuereinheit 20 ab und wird dort für eine sogenannte λ-Regelung herangezogen. Über die λ-Regelung werden in an sich bekannter Weise die Verbrennungsparameter wie Einspritzmenge, Öffnungsund Schließzeitpunkte der Gaswechselventile 2, ggf. Zündzeitpunkte etc. in Abhängigkeit der aktuellen (Last-) Anforderungen geregelt. Die Regelgröße ist hierbei der gemessene Sauerstoffgehalt KO.

Üblicherweise ist das Magnetventil 8 im nicht aktivierten Zustand offen, so dass die Hydraulikleitung 10 zum Druckraum 14 hin frei ist. Im aktivierten Zustand, wenn also das Magnetventil 8 mit einem ausreichenden Erregerstrom I beaufschlagt wird, befindet sich das Magnetventil 8 in seiner Schließstellung. Das Magnetventil 8 weist hierbei einen an sich bekannten und typischen Aufbau auf. Ein Anker wird in Schließ- bzw. Öffnungsrichtung durch den durch eine Elektrospule gebildeten Magneten betätigt. An diesem Anker ist ein Verschlusselement zum Verschließen der Hydraulikleitung 10 angeordnet. Üblicherweise wirkt die Magnetkraft gegen eine Federkraft einer im Magnetventil 8 angebrachten Feder, die das Magnetventil 8 im nicht aktiven Zustand in seine Ausgangslage, insbesondere seine Offenstellung drückt.

Der Erregerstrom I zeigt einen typischen Verlauf wie er in Fig. 2 dargestellt ist. Üblicherweise wird die Spule zunächst mit einem Einschaltstrom I₁ beaufschlagt, der sich zu einer Zeit t₁ einstellt. Dieser Einschaltstrom I₁ führt lediglich zu einer Vormagnetisierung, nicht jedoch zu einer Bewegung des Verschlusselements. Zum Aktivieren, also Schließen des Ventils 8 wird dieses mit einem Schließstrom I₂ beaufschlagt, der sich zum Zeitpunkt t₂ einstellt. Zu diesem Zeitpunkt bewegt sich das Verschlusselement in seine Schließstellung. Nach dem Schließen wird zu einem Zeitpunkt t₃ der Strom üblicherweise auf einen Haltestrom I₃ reduziert, der größer als der Einschaltstrom I₁ ist. Zu einem Zeitpunkt t₄ wird die Stromzufuhr abgeschaltet und der Erregerstrom I fällt ab. Der Zeitpunkt t₄ wird von der Steuereinheit 20 in Abhängigkeit der aktuellen Anforderungen vorgegeben.

In Fig. 2 ist ergänzend zu dem Erregerstrom I auch der diesem zugeordnete Verlauf des Hubs H des Gaswechselventils 2 im Zeitverlauf aufgetragen. Die gestrichelte Linie gibt eine Hüllkurve h wieder, die die Hubbewegung des Gaswechselventils 2 bei dauerhaft geschlossenem Magnetventil wiedergibt. Die Hüllkurve h entspricht daher der Bewegung des Gaswechselventils 2, wenn dieses zwangsweise der Bewegung des Nockens 6 unmittelbar folgt.

Durch das Abschalten des Erregerstroms I zum Zeitpunkt t₄ weicht die Hubbewegung des Gaswechselventils 2 von der Hüllkurve h ab. Das Gaswechselventil 2 schließt zu einem früheren Zeitpunkt. Der tatsächliche Verlauf der Hubbewegung des Gaswechselventils 2 bei dem in Fig. 2 dargestellten Verlauf des Erregerstroms I ist durch die durchgezogene Linie dargestellt. Wie zu erkennen ist, weicht nach einer mit der Hüllkurve h identischen Anfangsphase der Verlauf der Hubbewegung von der Hüllkurve h ab. Die abfallende Bewegung, also das Schließen des Gaswechselventils 2, wird vorliegend als ballistische Phase bezeichnet, da in diesem Zustand das Gaswechselventil 2 allein aufgrund der Federkraft in die Schließstellung zurückgeführt wird. Die Federkraft arbeitet hierbei entgegen der systembedingten Reibungskräfte. Diese sind maßgeblich auch durch das Motoröl und dessen Viskosität η bedingt. Die ballistische Phase kann hierbei in zwei Teilbereiche b1, b2 unterteilt werden. Die erste Teilphase b1 ist bedingt durch eine (ballistische) Schließbewegung des Magnetventils 8, für das die gleichen Überlegungen wie für das Gaswechselventil 2 gelten. Auch hier erfolgt der Verstellung des Ventils federkraftbetätigt gegen die maßgeblich durch die Viskosität bedingte Reibungskraft. Die zweite ballistische Teilphase b2 ist dann allein durch das Gaswechselventil 2 bedingt. Das Magnetventil 8 befindet sich zum Zeitpunkt t₅ in seiner geschlossenen Position.

Die Fläche unter der Kurve für die Hubbewegung des Gaswechselventils 2 korreliert zu der Menge der für einen Verbrennungstakt angesaugten Luft und bestimmt somit - bei definierter Einspritzmenge des eingespritzten Brennstoffs - das Mischungsverhältnis zwischen Brennstoff und Luft. Damit wird zugleich auch der Sauerstoffgehalt KO im Abgas beeinflusst.

Untersuchungen haben nunmehr gezeigt, dass der Verlauf der ballistischen Phase b1,b2 von der Viskosität η des eingesetzten Motoröls abhängig ist. Bei höherer Viskosität verschiebt sich die ballistische Phase b1,b2 nach rechts, d.h. das Gaswechselventil 2 schließt langsamer. Der Grund hierfür ist in der durch die höhere Viskosität bedingten höheren Reibungskraft zu sehen.

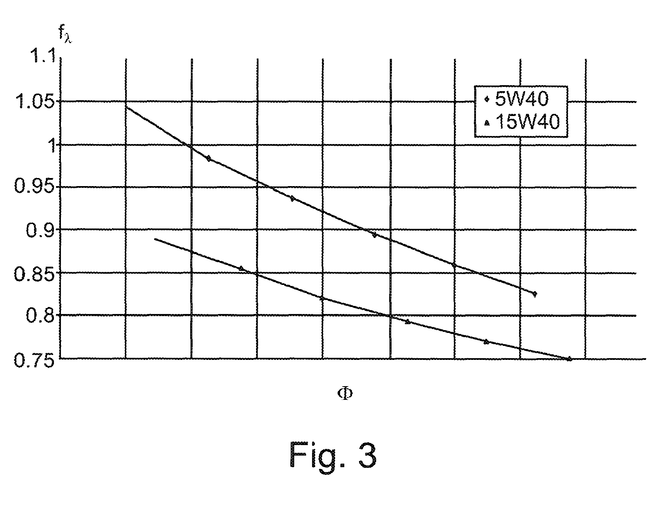

Fig. 3 zeigt hierzu beispielhaft das Ergebnis einer Testreihe, bei dem für zwei unterschiedliche Motoröle, nämlich eines gemäß der SAE-Klassifikation 5W40 und eines gemäß der SAE-Klassifikation 15W40 die Schließzeitpunkte t₄ des Magnetventils 8 variiert wurden. Die Schließzeitpunkte t₄ wurden hierbei in Schritten von 2,5° des Phasenwinkels φ variiert. Der Phasenwinkel φ ist eine elektrische Steuergröße, die das Abschalten der Bestromung des Magnetventils 8 und folglich den Schließzeitpunkt des Gaswechselventils 2 bestimmt. Er ist daher ein Maß für die Phasenlage zwischen der Schließstellung des Gaswechselventils und einer Drehposition der Kurbelwelle. Mit zunehmendem φ - Wert ist das Gaswechselventil 8 länger geöffnet und es gelangt mehr Sauerstoff in den Brennraum.

Auf der y-Achse ist ein Korrekturfaktor f_{λ} aufgetragen, der sich ergibt aus einer Abweichung (Verhältnis) des tatsächlichen λ-Wertes zum λ-Soll-Wert. Bei f_{λ} = 1 entspricht der tatsächliche λ-Wert dem λ-Soll-Wert. Allgemein gilt, dass bei einem zu großen λ-Wert die Verbrennung zu mager und bei zu kleinem λ-Wert die Verbrennung zu fett ist. Der λ-Wert wird in an sich bekannter Weise mittels der λ-Sonde gemessen und ist ein Maß für die Restsauerstoffkonzentration KO im Abgas Über die übliche λ-Regelung wird in Abhängigkeit der aktuellen Betriebsanforderungen ein gewünschter λ-Wert eingestellt. Regelparameter ist dabei üblicherweise die eingespritzte Kraftstoffmenge.

Bei den Untersuchungen wurde der Phasenwinkel φ bei unterschiedlichen vorgegebenen Motorparameter-Werten und unterschiedlichen Ölqualitäten sukzessive variiert. Die wesentlichen Parameter hierbei waren die Drehzahl des Motors, die Gaspedalposition sowie die Motoröltemperatur.

Wie insbesondere auch aus Fig. 3 hervorgeht, zeigte sich ein deutlicher Einfluss der Ölqualität und damit der Ölviskosität η auf den Korrekturfaktor f_{λ}.

Bei den Untersuchungen hat sich gezeigt, dass in Abhängigkeit des eingesetzten Öls der Korrekturfaktor f_{λ} variiert, dass also die Abweichung vom Soll-λ-Wert ölabhängig ist. Der gemessene Rest-Sauerstoffgehalt KO weicht daher von dem Soll-Sauerstoffgehalt, wie er über die λ-Regelung eingestellt werden soll, ab. Allgemein gilt, dass die Abweichung vom Soll-λ-Wert mit zunehmender Abweichung der tatsächlichen Viskosität von einer hinterlegten nominalen Viskositätskurve zunimmt. Ein abnehmender Korrekturfaktor f_{λ} bedeutet eine Zunahme des λ-Werts in Richtung mager und umgekehrt.

Ausgehend von diesen grundlegenden Erkenntnissen ist es daher vorgesehen, in der Steuereinrichtung 20 eine Analyseeinheit zu integrieren, die basierend auf der im laufenden Betrieb über den λ-Sensor gemessenen Sauerstoffkonzentration KO Rückschlüsse auf die aktuelle Viskosität η des eingesetzten Motoröls zieht. Hierbei ist wahlweise die direkte Auswertung der gemessenen Sauerstoffkonzentration KO oder auch eine Auswertung und Analyse des Steuersignals der λ-Regelung vorgesehen. Wie sich nämlich aus Fig. 3 ergibt, entsteht ein Korrekturbedarf, sobald die tatsächliche Viskosität von einer in der Steuereinheit 20 hinterlegten (z.B. temperaturabhängigen) Soll-Viskosität abweicht. Diese Viskositätsabweichung führt daher zu einem geänderten Regelverhalten der λ-Regelung, welches ausgewertet werden kann.

Vorzugsweise sind hierzu für unterschiedliche Parametersätze eine Vielzahl von Kennlinienfeldern hinterlegt. Derartige Kennlinienfelder geben beispielsweise - ähnlich wie in Fig. 3 dargestellt - eine Korrelation zwischen dem Korrekturfaktor f_{λ} und dem elektrischen Phasenwinkel φ für unterschiedliche Motorölvarianten jeweils bei einem definierten Arbeitspunkt des Motors an. Unter Arbeitspunkt des Motors wird verstanden, dass die den Verbrennungsvorgang charakterisierenden Parameter, wie beispielsweise Motordrehzahl, Gaspedalstellung, Öltemperatur, etc. einen festen definierten Wert haben.

Bei der Analyse wird dann vorzugsweise durch einen einfachen Vergleich der gemessenen Werte mit den hinterlegten Kennlinienfeldern Rückschlüsse auf die Viskosität und Qualität des eingesetzten Öls gezogen. Diese Erkenntnis wird dann vorzugsweise herangezogen, um wahlweise oder in Kombination
- ein Maß für die eingesetzte Ölqualität zu ermitteln, insbesondere zu ermitteln, welcher SAE-Klassifikation das eingesetzte Motoröl zuzuordnen ist,
- unzulässige Motoröle zu erkennen,
- eine abrupte Änderung des Motoröls beispielsweise im Nachgang zu einem Motorölwechsel zu erkennen und insbesondere in der Steuereinheit 20 eine Markierung zu setzen,
- anhand der gesetzten Markierung oder auch anhand der aktuell bestimmten Ölqualität den Zeitpunkt für einen Ölwechsel zu bestimmen und gegebenenfalls anzuzeigen,
- das eingesetzte Motoröl auf Änderungen im Hinblick auf seine Eigenschaften (Alterung) zu überwachen,
- die gewonnenen Erkenntnisse über das eingesetzte Motoröl und seine Auswirkungen auf die ballistische Phase unmittelbar für die Steuerung der Gaswechselventile 2, insbesondere für die λ-Regelung zu berücksichtigen und heranzuziehen, um beispielsweise den Schließzeitpunkt t₄ des Magnetventils 8 festzulegen, sowie
- die gesamte Steuerung des Gaswechselventils 2 in Abhängigkeit der ermittelten Ölqualität bzw. in Abhängigkeit der ermittelten Viskositätseigenschaften des Öles vorzunehmen.

Im Vergleich zu der bisherigen Steuerung, in Abhängigkeit der aktuell gemessenen Öltemperatur, hat der letztgenannte Punkt den entscheidenden Vorteil, dass die aktuell tatsächlichen Eigenschaften des Motoröls für die Steuerung herangezogen werden, so dass automatisch beispielsweise Alterungseffekte berücksichtigt werden.

Von besonderem Vorteil bei diesem Verfahren ist die Tatsache, dass zusätzliche Hardware-Komponenten neben den bereits vorhandenen Komponenten im Kraftfahrzeug, wie beispielsweise die λ-Sonde, nicht erforderlich und auch nicht vorgesehen sind. Die Auswertung und Analyse erfolgt allein aufgrund sowieso vorhandender Messdaten.

### Liste der Bezugszahlen

- 2: Gaswechselventil
- 4: Kraftfahrzeug
- 6: Nocken
- 7: Nockenwelle
- 8: Magnetventil
- 10: Hydraulikleitung
- 12: Hydraulikzylinder
- 14: Druckraum
- 16: Feder
- 18: Kolben
- 20: Steuereinheit
- 22: λ-Sonde

- b1,b2: ballistische Phasen
- η: Viskosität
- h: Hüllkurve
- f_{λ}: Korrekturfaktor
- KO: Sauerstoffgehalt
- S(KO): Sauerstoff-Messsignal
- I: Erregerstrom

## Patentansprüche

1. Verfahren zur Ermittlung einer Viskositäts-Kenngröße eines Motoröls in einem Verbrennungsmotor mit einer hydraulischen Steuerung der Gaswechselventile (2), **dadurch gekennzeichnet, dass** eine Vergleichsgröße, die mit einer gemessenen Sauerstoffkonzentration (KO) in einem Abgas des Verbrennungsmotors korreliert ist, als ein Maß für die Viskositäts-Kenngröße, insbesondere die Viskosität (η) herangezogen wird.

2. Verfahren nach Anspruch 1, **dadurch gekennzeichnet, dass** die gemessene Sauerstoffkonzentration (KO) für eine λ-Regelung herangezogen wird und die λ-Regelung zur Ermittlung der Viskositäts-Kenngröße ausgewertet wird.

3. Verfahren nach Anspruch 1 oder 2, **dadurch gekennzeichnet, dass** eine von der λ-Regelung vorgenommene Korrektur der Sauerstoffkonzentration (KO) zur Ermittlung der Viskositäts-Kenngröße ausgewertet wird.

4. Verfahren nach einem der vorhergehenden Ansprüche, **dadurch gekennzeichnet, dass** in einer Steuereinrichtung (20) eine Korrelation zwischen der Vergleichsgröße und der Viskositäts-Kenngröße hinterlegt ist.

5. Verfahren nach Anspruch 4, **dadurch gekennzeichnet, dass** die Korrelation in Abhängigkeit zumindest eines Betriebsparameters des Verbrennungsmotors wie Temperatur oder Motordrehzahl hinterlegt ist.

6. Verfahren nach Anspruch 4 oder 5, **dadurch gekennzeichnet, dass** die Korrelation in Abhängigkeit einer Kenngröße des Motoröls, wie eine Viskositätsklasse, hinterlegt ist.

7. Verfahren nach einem der vorhergehenden Ansprüche, **dadurch gekennzeichnet, dass** der Verbrennungsmotor mehrere Zylinder aufweist und zylinderspezifisch die Viskositäts-Kenngröße ermittelt wird.

8. Verfahren nach einem der vorhergehenden Ansprüche, **dadurch gekennzeichnet, dass** die ermittelte Viskositäts-Kenngröße ausgewertet und wahlweise oder in Kombination herangezogen wird für
- eine Ermittlung eines Maßes für eine Ölqualität
- eine Erkennung von unzulässigen Motorölen
- eine Erkennung und Auswertung einer abrupten Änderung der Viskositäts-Kenngröße
- eine Bestimmung eines Ölwechselintervalls
- die Steuerung der Gaswechselventile (2).

9. Steuereinrichtung (20) zur Ermittlung einer Viskositäts-Kenngröße eines Motoröls in einem Verbrennungsmotor mit einer hydraulischen Steuerung der Gaswechselventile (2), **dadurch gekennzeichnet, dass** die Steuereinrichtung (20) derart ausgebildet ist, dass eine Vergleichsgröße, die mit einer gemessenen Sauerstoffkonzentration (KO) in einem Abgas des Verbrennungsmotors korreliert ist, als ein Maß für die Viskositäts-Kenngröße der Hydraulikflüssigkeit, insbesondere die Viskosität (η) herangezogen wird.

## Claims

1. Method for determining a viscosity parameter of an engine oil in an internal combustion engine with hydraulic control of the charge-cycle valves (2), **characterized in that** a comparison variable, which is correlated with a measured oxygen concentration (KO) in an exhaust gas of the internal combustion engine, is used as a measure of the viscosity parameter, in particular the viscosity (η).

2. Method according to Claim 1, **characterized in that** the measured oxygen concentration (KO) is used for a λ control process, and the λ control process is evaluated in order to determine the viscosity parameter.

3. Method according to Claim 1 or 2, **characterized in that** a correction of the oxygen concentration (KO) which is performed by the λ control process is evaluated in order to determine the viscosity parameter.

4. Method according to one of the preceding claims, **characterized in that** a correlation between the comparison variable and the viscosity parameter is stored in a control device (20).

5. Method according to Claim 4, **characterized in that** the correlation is stored as a function of at least one operating parameter of the internal combustion engine such as the temperature or engine speed.

6. Method according to Claim 4 or 5, **characterized in that** the correlation is stored as a function of a parameter of the engine oil such as a viscosity class.

7. Method according to one of the preceding claims, **characterized in that** the internal combustion engine has a plurality of cylinders, and the viscosity parameter is determined on a cylinder-specific basis.

8. Method according to one of the preceding claims, **characterized in that** the viscosity parameter which is determined is evaluated and is used optionally or in combination for
- determining a measure of oil quality,
- detecting unacceptable engine oils,
- detecting and evaluating an abrupt change in the viscosity parameter,
- determining an oil change interval, and
- controlling the charge-cycle valves (2).

9. Control device (20) for determining a viscosity parameter of an engine oil in an internal combustion engine having a hydraulic control of the charge-cycle valves (2), **characterized in that** the control device (20) is embodied in such a way that a comparison variable, which is correlated with a measured oxygen concentration (KO) in an exhaust gas of the internal combustion engine, is used as a measure for the viscosity parameter of the hydraulic fluid, in particular the viscosity (η).

## Revendications

1. Procédé de détermination d'une grandeur caractéristique de la viscosité d'une huile moteur dans un moteur à combustion interne doté d'une commande hydraulique des soupapes (2) de remplacement des gaz, **caractérisé en ce que**
une grandeur de comparaison corrélée à la concentration en oxygène (KO) mesurée dans les gaz d'échappement du moteur à combustion interne est utilisée comme mesure de la grandeur caractéristique de la viscosité et en particulier de la viscosité (η).

2. Procédé selon la revendication 1, **caractérisé en ce que** la concentration en oxygène (KO) mesurée est utilisée pour une régulation du λ et **en ce que** la régulation du λ est évaluée pour déterminer la grandeur caractéristique de la viscosité.

3. Procédé selon les revendications 1 ou 2, **caractérisé en ce qu'**une correction de la concentration en oxygène (KO) réalisée par la régulation du λ est évaluée pour déterminer la grandeur caractéristique de la viscosité.

4. Procédé selon l'une des revendications précédentes, **caractérisé en ce qu'**une corrélation entre la grandeur de comparaison et la grandeur caractéristique de la viscosité est conservée dans un dispositif de commande (20).

5. Procédé selon la revendication 4, **caractérisé en ce que** la corrélation est conservée en fonction d'au moins un paramètre de fonctionnement du moteur à combustion interne, par exemple sa température ou le régime moteur.

6. Procédé selon les revendications 4 ou 5, **caractérisé en ce que** la corrélation est conservée en fonction d'une grandeur caractéristique de l'huile moteur, par exemple une classe de viscosité.

7. Procédé selon l'une des revendications précédentes, **caractérisé en ce que** le moteur à combustion interne présente plusieurs cylindres et **en ce que** la grandeur caractéristique de viscosité est déterminée cylindre par cylindre.

8. Procédé selon l'une des revendications précédentes, **caractérisé en ce que** la grandeur caractéristique de la viscosité qui a été déterminée est évaluée et est utilisée sélectivement ou en combinaison pour :
- déterminer une mesure de la qualité de l'huile,
- détecter des huiles moteur inadmissibles,
- détecter et évaluer une modification brusque de la grandeur caractéristique de la viscosité,
- déterminer un intervalle entre les vidanges d'huile et
- commander les soupapes (2) de remplacement des gaz.

9. Dispositif de commande (20) destiné à déterminer une grandeur caractéristique de la viscosité d'une huile moteur dans un moteur à combustion interne doté d'une commande hydraulique de soupape (2) de remplacement de gaz,
**caractérisé en ce que**
le dispositif de commande (20) est configuré de telle sorte qu'une grandeur de comparaison corrélée à la concentration en oxygène (KO) mesurée dans les gaz d'échappement du moteur à combustion interne est utilisée comme mesure de la grandeur caractéristique de la viscosité du fluide hydraulique et en particulier de sa viscosité (η).
